(19) Europäisches Patentamt
European Patent Office
Office européen des brevets

(11) Veröffentlichungsnummer: **0 274 127 B1**

(12) **EUROPÄISCHE PATENTSCHRIFT**

(45) Veröffentlichungstag der Patentschrift: **01.12.93**

(51) Int. Cl.5: **C08G 69/08**, C08G 69/04, A61K 9/52

(21) Anmeldenummer: **87119291.0**

(22) Anmeldetag: **29.12.87**

# (54) Biologisch abbaubare Poly-(hydroxyalkyl)-Aminodicarbonsäure-Derivate, Verfahren zu ihrer Herstellung und Verwendung derselben für Depotzubereitungen mit kontrollierter Wirkstoffabgabe.

(30) Priorität: **03.01.87 DE 3700128**

(43) Veröffentlichungstag der Anmeldung:
**13.07.88 Patentblatt 88/28**

(45) Bekanntmachung des Hinweises auf die Patenterteilung:
**01.12.93 Patentblatt 93/48**

(84) Benannte Vertragsstaaten:
**AT BE CH DE FR GB IT LI LU NL**

(56) Entgegenhaltungen:
**EP-A- 0 130 935**
**EP-A- 0 179 023**
**DE-A- 3 612 102**
**US-A- 4 356 166**

(73) Patentinhaber: **HOECHST AKTIENGESELLSCHAFT**

**D-65926 Frankfurt(DE)**

(72) Erfinder: **Bader, Hubert, Dr.**
**Im Münchfeld 23**
**D-6500 Mainz(DE)**
Erfinder: **Rüppel, Diether, Dr.**
**Karl-König-Weg 1**
**D-6230 Frankfurt am Main 80(DE)**
Erfinder: **Walch, Axel, Dr.**
**Hans-Sachs-Strasse 5**
**D-6000 Frankfurt am Main 90(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft biologisch abbaubare Poly-(hydroxyalkyl)-Aminodicarbonsäure-Derivate, ein Verfahren zu ihrer Herstellung und die Verwendung derselben für Depotzubereitungen mit kontrollierter Wirkstoffabgabe. Dabei werden die Wirkstoffe in einer Matrix, bestehend aus den erfindungsgemäßen Polyamiden, eingebettet und in vivo durch Bioerosion der Matrix kontrolliert freigesetzt. Beim Abbau der erfindungsgemäßen Produkte entstehen ausschließlich körpereigene, bzw. in ihrer Bioverträglichkeit bekannte Fragmente, die in natürlichen Stoffwechselwegen metabolisiert oder auf Grund ihrer Wasserlöslichkeit durch die Nieren ausgeschieden werden.

Eine moderne Arzneitherapie erfordert insbesondere zur Applikation von Wirkstoffen neue Darreichungsformen, die eine kontrollierte Abgaberate der Wirkstoffe mit hoher Biokompatibilität des Depots vereinigen. Eine langandauernde kontrollierte Wirkstoffabgabe ist wegen der zunehmenden Bedeutung chronischer Erkrankungen und langzeitorientierter Therapiekonzepte in Human- und Veterinärmedizin von großer Aktualität. Als Matrixmaterialien für solche Depotsysteme sind bioabbaubare Polymere besonders vorteilhaft, da die Bioerosion die Wirkstofffreisetzung steuert und die chirurgische Entfernung eines derartigen Depots entbehrlich macht.

Arzneiabgabesysteme, in denen der Wirkstoff in einer nicht abbaubaren Polymermatrix dispergiert ist und durch Diffusion freigesetzt wird, sind in der amerikanischen Patentschrift 4,069,307 beschrieben. Nach Erschöpfung des Wirkstoffreservoirs müssen solche Implantate jedoch operativ aus dem Organismus entfernt werden.

In biologisch abbaubaren Arzneistoffabgabesystemen, wie in dem amerikanischen Patent 4,093,709 angegeben, wird der Wirkstoff in einem bioabbaubaren Polymeren dispergiert, welches beim Abbau den Wirkstoff freigibt. Typische, nach dem Stand der Technik meist untersuchte, biologisch abbaubare Polymere sind Homo- und Copolyester, insbesondere der Milch- und Glykolsäure, wie sie in den US-Patenten 3,773,919 bzw. 3,297,033 beschrieben sind. Nachteilig ist u.a. die geringe oder schlecht kontrollierbare Quellbarkeit der Polyester im physiologischen Milieu, welche den Transport der im Implantat inkorporierten Wirkstoffe durch die Polymermatrix hindurch an die Oberfläche behindert und eine nach initialem "burst effect" nur geringe Freisetzungsrate bewirkt.

Die Europäische Patentanmeldung mit der Veröffentlichungs Nr. 0 130 935 beschreibt biologisch abbaubare Polypeptide, die Monomereneinheiten von Asparaginsäure und Glutaminosäureester enthalten.

In neuerer Zeit sind Polyacetale und -ketale (US-PS 4,304,767) bzw. Polyanhydride (H.G. Rosen et al., Biomaterials 4, 131 (1983)) und Polyorthoester (US-PS 4,180,646) beschrieben worden, die als biologisch abbaubare Polymere für den Einsatz als Implantatmaterialien entwickelt wurden.

Der Abbau dieser Polymere ist, ähnlich den angeführten Polyestern, nur durch die hydrolytische Beständigkeit der Carbonylfunktion in der Polymer-Hauptkette bestimmt und läßt sich lediglich durch Comonomereneinbau geringfügig beeinflussen. Solche Polymere verfügen außerdem nicht über eine für Implantationszeiträume von Monaten ausreichende Stabilität.

Als weitere Polymerklasse sind in der amerikanischen Patentschrift 3,371,069 Polyamide, insbesondere Poly-α-L-Aminosäuren, als bioresorbierbare Implantatmaterialien beschrieben worden. Die technische Herstellung von Polyaminosäuren erfordert jedoch den Einsatz teurer geschützter Aminosäuren, größere Mengen an hochgiftigem Phosgen, die Abspaltung der Schutzgruppen und die anschließende Derivatisierung der erhaltenen Polymere.

Ein weiterer Nachteil derartiger Polyamide ist das Vorliegen geladener Gruppen durch nicht vollständige Derivatisierung beim Aufbau des Implantatmaterials und die zusätzliche Erzeugung solcher ionogener Gruppen durch die Bioerosion im Organismus. Die in den Patentschriften erwähnte reine Poly-L-Glutaminsäure sowie reines Poly-L-Lysin sind toxikologisch äußerst bedenklich (A.D. Kenny, Proc. Soc. Exp. Biol. Med. 100, 778 (1959)) und ihre durch Bioabbau aus hydrophoben Derivaten entstehenden Copolymere müssen daher ebenfalls sehr kritisch bewertet werden.

In der US-PS 4 356 166 werden bioabbaubare Implantatmaterialien beschrieben, die in vivo eine bioaktive Verbindung freisetzen. Als bioaktive Verbindungen werden in der US-PS 4 356 166 Progestine beschrieben, die zunächst chlorformyliert und dann kovalent an das Polymer gebunden werden. Als Polymere werden dabei Poly-(hydroxyalkyl)-L-glutamin oder Poly(hydroxyalkyl)-L-aspartamid eingesetzt. Die bioaktiven Verbindungen sind entweder über eine sogenannte "Spacer-Group" oder aber direkt über die reaktive Komponente des Polymeren gebunden. Die Freigaberate der bioaktiven Verbindung wird über das Molekulargewicht des Polymeren oder über die Länge und den Charakter der "Spacer-Group" gesteuert.

Nachteilig an den Substanzen gemäß US-PS 4 356 166 ist, daß sie bereits selbst Pharmaka mit hoher pharmakologischer Aktivität darstellen. In solchen Polymer/Wirkstoff-Konjugaten (polymeric drugs) bilden biokompatibles Polymer und Wirkstoff eine Einheit, deren Eigenschaften in komplexer Weise von beiden

Komponenten bestimmt werden. Die Freigaberate des polymerfixierten bioaktiven Moleküls ist im Rahmen der vorgenannten Parameter variabel, jedoch entscheidend von der Natur des Wirkstoffs abhängig. Hydrophobe bioaktive Stoffe, wie z.B. Steroidhormone, können im wäßrigen biologischen Milieu nur sehr langsam vom Polymerrückgrat abgespalten werden und eignen sich daher ausschließlich für extreme Langzeitdepotformen. Für jeden Wirkstoff müssen neue Polymer/Wirkstoff-Konjugate synthetisiert werden, was die Verwendbarkeit des in der US-PS 4 356 166 beschriebenen Konzeptes polymerfixierter Pharmaka außerordentlich eingeschränkt.

Aus diesen Gründen eignen sich diese Substanzen nicht für den Einsatz als gesteuert abbaubare Polymere, die durch ihre eigene Biodegradation einen Wirkstoff freisetzen, der in die inerte Polymermatrix eingebettet ist, ohne dabei chemisch an das Polymere gebunden zu sein.

Es wurden nun Poly-(hydroxyalkyl)-Aminodicarbonsäure-Derivate synthetisiert, die sich überraschenderweise hervorragend zum Einsatz als abbaubare Arzneistoffimplantate mit kontrollierter Wirkstoffabgabe eignen. Erfindungswesentlich ist dabei, daß die Wirkstoffe nicht chemisch an das Polymere gebunden werden, sondern lediglich in diese Polymermatrix eingebettet werden. Durch Einbau geeigneter biologisch inaktiver Acylgruppen läßt sich die Abbaugeschwindigkeit des Polymers in vivo im gewünschter Weise steuern und somit gleichzeitig die Freigaberate des Wirkstoffs. Der Vorteil dieser Verfahrensweise ist, daß nun auch solche Wirkstoffe über einen längeren Zeitraum mit relative konstanter Dosis appliziert werden können, die entweder überhaupt nicht chemisch an ein Polymer gebunden werden können, oder aber zu empfindlich sind, um die doch recht drastischen Bedingungen bei der chemischen Ankoppelung an das Polymer zu überstehen. Darüber hinaus sind die Polymere als pharmakologisch inerte Matrix grundsätzlich für alle relevanten Pharmaka universell einsetzbar, unabhängig von der Molekülgröße und anderer physikalisch-chemischer Parameter. Diese biologisch abbaubaren Polymere erhält man durch Polykondensation von Aminodicarbonsäuren, die anschließend mit Aminoalkoholen zu Poly(hydroxyalkyl)-Aminodicarbonsäuren umgesetzt werden und dann mit Carbonsäuren, Carbonsäurehalogeniden oder Halogenameisensäureestern in einer polymeranalogen Acylierung zu den gewünschten Poly-(hydroxyalkyl)-Aminodicarbonsäure-Derivaten reagieren. In vivo werden diese Polymere zu untoxischen, nicht allergenen und nicht immunogenen Verbindungen metabolisiert und ausgeschieden.

Die Erfindung betrifft somit:

Poly-(hydroxyalkyl)-Aminodicarbonsäure-Derivate der Formel I

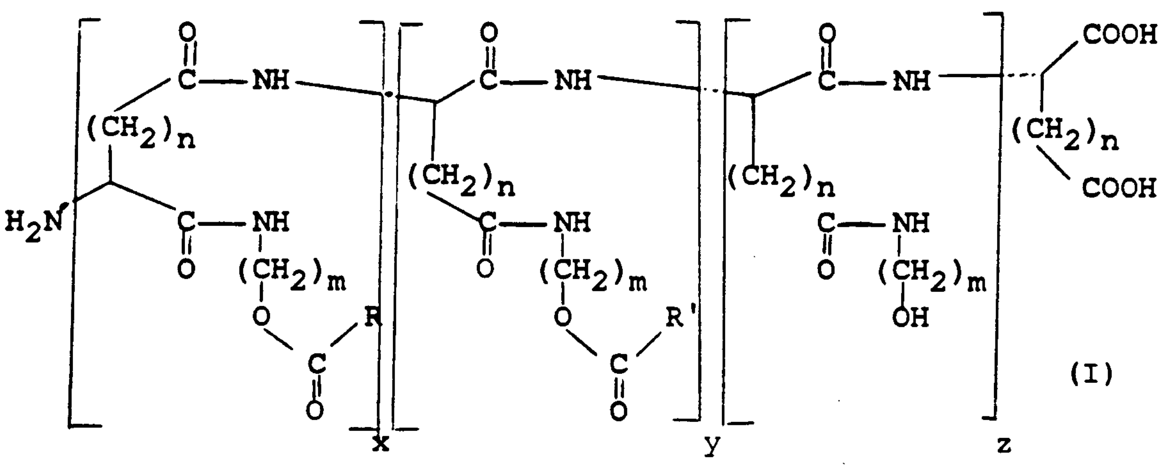

(I)

in denen n 1 oder 2, m 2 bis 6, x und y 1 bis 400 und z 0 bis 40 sind, und die Summe von x, y und z 2 bis 840 ist und der Anteil der Hydroxyalkylamino-dicarbonsäureamid-Monomereinheit bezogen auf den molaren Anteil den acylierten Aminodicarbonsäureamide nicht mehr als 30 % ist, dadurch gekennzeichnet, daß die Reste R und R' gleich oder unabhängig voneinander verschieden sind und verzweigtes oder unverzweigtes, gesättigtes Alkyl, Cycloalkyl, Alkyloxy oder Cycloalkyloxy mit insgesamt 1 - 22 C-Atomen im Alkylteil, wobei der Alkylteil gegebenenfalls durch eine Carbonyloxy-Gruppe unterbrochen sein kann, oder biologisch inaktive Steroidalkohole, gebunden über ihre Hydroxylgruppen, bedeutet, wobei die in eckige Klammern gesetzten Monomereinheiten statistisch im Polymeren verteilt sind.

Ebenso betrifft die Erfindung das Verfahren zur Herstellung der obengenannten Polyamide und deren Verwendung auch in Abmischungen mit anderen, bioverträglichen Polyamiden, besonders in Verbindung mit biologisch aktiven Substanzen, als abbaubare Wirkstoffdepotzubereitung mit kontrollierter Wirkstoffabgabe.

Im folgenden wird die Erfindung detailliert beschrieben.

Als Aminodicarbonsäuren können Asparaginsäure (n = 1) oder Glutaminsäure (n = 2) eingesetzt werden.

Bevorzugt wird Asparaginsäure eingesetzt, die in einer Polykondensationsreaktion zu der entsprechenden Polyanhydroasparaginsäure (VII) reagiert. Durch Umsetzung mit einem Aminoalkohol der Formel IV

$H_2N-(CH_2)_m-OH$ IV

in dem m eine Zahl von 2 bis 6 ist, wobei 3-Aminopropanol, insbesondere 2-Aminoethanol bevorzugt ist, erhält man α,β-Poly-(hydroxyalkyl)-DL-Aspartamid der Formel II

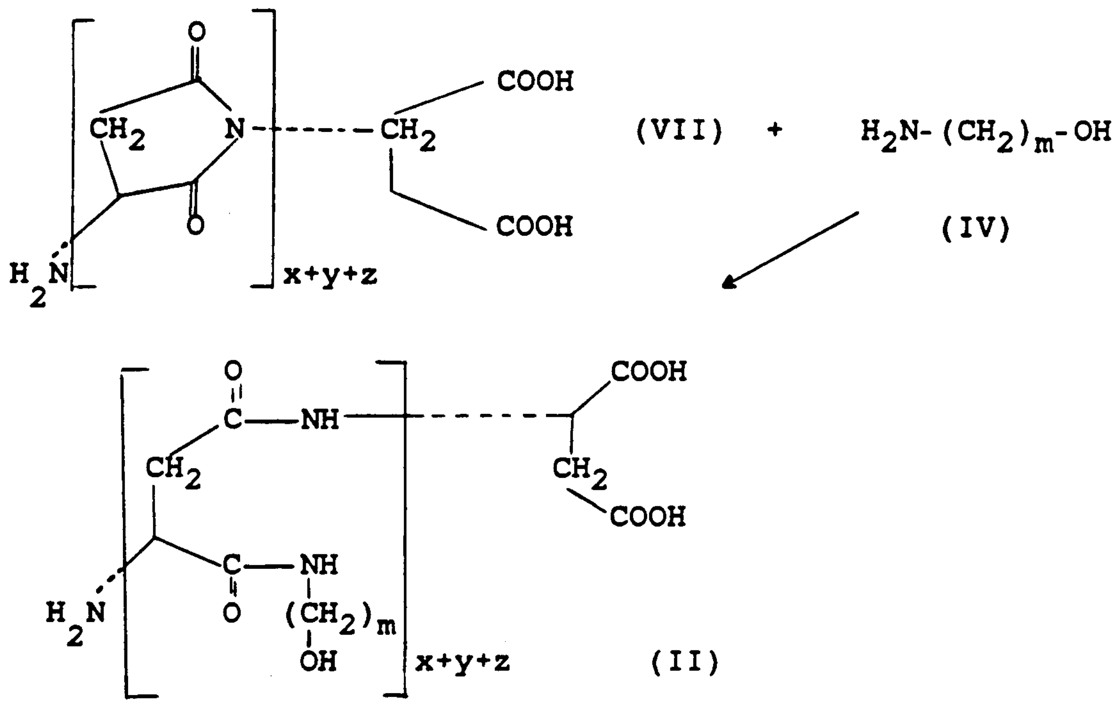

(VII) +

(IV)

(II)

Ein Verfahren zur Herstellung von α,β-Poly-(2-hydroxyethyl)-DL-Aspartamid (PHEA) wird von P. Neri, G. Antoni, F. Benvenuti, F. Cocola, G. Gazzei, in J. Med. Chem. 16, 893 (1973) beschrieben. Eine allgemeine Arbeitsvorschrift zur Herstellung von PHEA findet sich in P. Neri, G. Antoni, Macromol. Synth. 8, 25. Auf diese Literaturstelle wird an dieser Stelle ausdrücklich Bezug genommen. Die Umsetzung erfolgt in hoher Ausbeute zu einem Produkt mit hohem Reinheitsgrad. In gleicher Weise lassen sich die analogen höheren Aminoalkoholderivate der Polyanhydroasparaginsäure herstellen.

Zur Herstellung von Poly-(hydroxyalkyl)-L-glutamin muß ein anderes, umständlicheres Verfahren angewendet werden, wie es in der US-PS 4 356 166 beschrieben ist. Dabei wird zunächst die γ-ständige COOH-Gruppe der L-Glutaminsäure durch Veresterung mit Benzylalkohol geschützt. Anschließend wird dieses γ-Benzyl-Glutamat mit Phosgen zu einem N-Carboxyanhydrid umgesetzt, welches dann nach Zugabe von Triethylamin in einem inerten Lösungsmittel polymerisiert, wobei man Poly-γ-(benzyl)-L-glutamat erhält. Die Abspaltung der Schutzgruppe erfolgt entweder durch Zugabe von einem HCl/HBr-Gemisch zur freien Poly-α-L-Glutaminsäure oder aber in Gegenwart von Hydroxyalkylaminen zu den analogen Poly-α-(hydroxyalkyl)-L-glutaminen. Eine allgemeine Arbeitsvorschrift zur Herstellung von Poly-α-(hydroxypropyl)-L-glutamin findet sich in der US-PS 4 356 166, auf die an dieser Stelle ausdrücklich Bezug genommen wird. In gleicher Weise lassen sich auch den analogen höherer Aminoalkoholderivate von Poly-γ-(benzyl)-L-glutamat herstellen.

Diese Poly-(hydroxyalkyl)-Aminodicarbonsäuren der Formel IIa

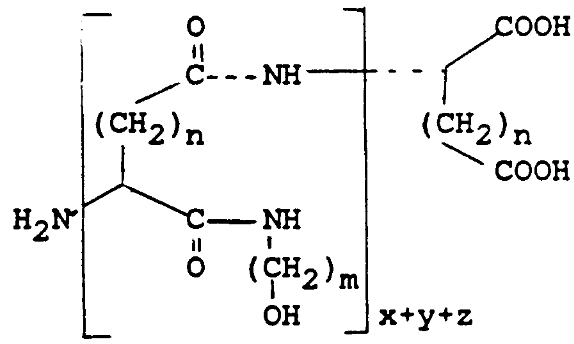

(IIa)

bevorzugt $\alpha,\beta$-Poly-(hydroxylalkyl)-DL-Aspartamid, werden nun erfindungsgemäß im folgenden Reaktionsschritt mit einer oder mehreren verschiedenen, biologisch inaktiven Verbindungen der Formel V und/oder VI

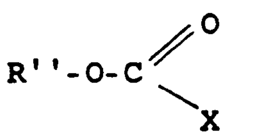

(V)

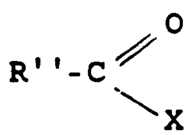

(VI)

zu den erfindungsgemäßen Poly-(hydroxyalkyl)-Aminodicarbonsäure-Derivaten umgesetzt. Hierbei steht X für eine Abgangsgruppe, die eine schonende Veresterung der Polymer-Alkoholgruppe ermöglicht. Bevorzugt sind Chlor, Brom, Jod, Imidazolide, Anhydride oder Hydroxyl, insbesondere Chlor. Geeignete Reste R″ sind Alkyl- oder Cycloalkylgruppen mit insgesamt 1 - 22 C-Atomen in den Alkylresten. Bevorzugt werden in Homopolymeren Alkylreste mit 5 - 22 C-Atomen, insbesondere solche mit 6 - 18 C-Atomen eingesetzt. Besonders bevorzugt in Verbindungen gemäß Formel V sind solche Alkylreste R″ mit gerader Kohlenstoffatomanzahl; in Verbindungen gemäß Formel VI solche Alkylreste R″ mit ungerader Kohlenstoffatomenzahl. Bei der Herstellung von Copolymeren, also bei der Umsetzung mit zwei oder mehreren verschiedenen Verbindungen der Formel V und/oder VI können auch Verbindungen mit kürzeren Alkylresten als in den Vorzugsbereichen angegeben, eingesetzt werden. So können beispielsweise 50 Mol-% einer Verbindung mit C-8-Alkylkette zusammen mit 50 Mol-% einer Verbindung mit C-2-Alkylkette zu den Poly-(hydroxyalkyl)-aminodicarbonsäurederivaten umgesetzt werden.
Die genannten Alkylreste können verzweigt, bevorzugt jedoch unverzweigt sein. Bevorzugt lassen sich gesättigte Alkylreste einsetzen. Weiterhin kann der Alkylrest auch durch eine Carbonyloxy-Gruppe unterbrochen sein. Als Reste R" sind weiterhin auch Steroide, insbesondere Cholesteryl geeignet.

Die Umsetzung mit den Verbindungen des Formeltyps V oder VI kann sowohl mit einer einzigen solchen Verbindung erfolgen als auch mit beliebigen Kombinationen dieser Verbindungen oder auch mit Verbindungen, die unterschiedliche, z.B. in der Art ihrer Verzweigung, insbesondere in ihrer Kettenlänge verschiedene Reste R" haben.

Die letztgenannte polymeranaloge Acylierung wird nach bekannten Verfahren der organischen Chemie durchgeführt, wie sie z.B. in Houben-Weyl, Methoden der Organischen Chemie, Bd. VIII (3, S. 543 ff., G. Thieme Verlag, Stuttgart, 1952) beschrieben sind. Sie verläuft selektiv an der Hydroxylfunktion zu Estern bzw. Carbonaten, ohne weitere Funktionen am Ausgangspolymeren anzugreifen. Besonders eignet sich die Einhorn-Variante der Schotten-Baumann-Acylierung in Gegenwart von Pyridin. Dabei werden unter schonenden Bedingungen sehr hohe Derivatisierungsgrade (größer 70 %) erzielt.

Bei Derivatisierungsgraden unter 100 %, d.h. bei noch vorhandenen freien Hydroxylgruppen, und für den Fall, daß verschiedene Substituenten R und R' in das Polymer eingebaut sind, kann das Polymer aus bis zu drei unterschiedlichen Monomereinheiten (in Formel I in eckige Klammern gesetzt) aufgebaut sein, die dann statistisch in diesem Polymeren verteilt sind.

Bei Acylierung mit nur einer Substanz gemäß Formel V oder VI und bei Derivatisierungsgraden von 100 % entsteht ein Homopolymer.

Das Molekulargewicht der erfindungsgemäßen Polymeren beträgt 200 bis 90000 (für $x+y+z=$ 2 bis 840 und bei einem Molekulargewicht von ca. 100), bevorzugt 20000 bis 84000 (für $x+y+z=$ 200 bis 840). Der Anteil der Hydroxyalkylaminodicarbonsäureamid-Monomereinheit - bezogen auf den molaren Anteil der

acylierten Aminodicarbonsäureamide - sollte 30 mol-%, bevorzugt 10 mol-% nicht überschreiten (d.h. z = 0 bis 40).

Unter den Carbonsäurehalogeniden und Halogenameisensäureestern sind die Chlorverbindungen bevorzugt.

Die bevorzugt als Ausgangssubstanzen eingesetzten Chlorameisensäureester erhält man durch Umsetzung von Phosgen mit den entsprechenden biologisch inaktiven, physiologisch unbedenklichen, aliphatischen oder cycloaliphatischen, insbesondere unverzweigten Alkoholen. Besonders bevorzugt werden solche Alkohole eingesetzt, die eine geradzahlige Kohlenstoffanzahl aufweisen. Auf diese Weise erhält man auch die chlorformylierten Steroide. Prinzipiell sind somit alle biologisch inaktiven Steroide zugänglich, die reaktive Hydroxylgruppen aufweisen. Beispielsweise seien hier genannt: Cholesterol, Cholestanol, Coprostanol, Ergosterol, Sitosterol oder Stigmasterol.

Die ebenfalls bevorzugt als Ausgangsverbindungen einsetzbaren Säurechloride erhält man beispielsweise aus den entsprechenden Carbonsäuren durch Umsetzung mit Phosphortrichlorid, Phosphorpentachlorid, Oxalylchlorid oder Thionylchlorid (Houben-Weyl, Meth. d. Org. Chem., 4. Auf., Bd. VIII, S. 463 ff., Thieme Verlag Stuttgart 1952; bzw. Houben-Weyl, Meth. d. Org. Chem. 4. Aufl., Erg. Bd. E5, S. 587 ff., Thieme Verlag Stuttgart, 1985).

Verbindungen des Formeltyps V oder VI, in denen eine Alkylkette durch eine Carbonyloxy-Gruppe unterbrochen ist, werden beispielsweise durch Umsetzung von cyclischen Dicarbonsäureanhydriden mit Alkoholen hergestellt. Die auf diese Weise erhaltenen Dicarbonsäuremonoester werden dann analog zu den oben beschriebenen Carbonsäuren, z.B. mit Oxalylchlorid zu den entsprechenden Säurechloriden umgesetzt.

Die Hydrophobie der Poly-(hydroxyalkyl)-Aminodicarbonsäure-Derivate - und damit die Verweildauer eines daraus hergestellten Implantats im Organismus - läßt sich sowohl über die Anzahl der C-Atome im Acylierungsagens als auch über den Substitutionsgrad in weiten Grenzen einstellen. So sind die entsprechenden Derivate mit mindestens 6 C-Atomen im Alkylteil schon wasserunlöslich.

Eine exakte Angabe der Relation Kettenlänge - Abbauzeit in vitro/vivo ist jedoch nur schwer möglich, da die Abbauzeit außer von der Kettenlänge noch von einer Vielzahl anderer Parameter abhängig ist; beispielsweise von der Partikelgröße und -verteilung, der Herstellungsmethode z.B. für Microspheres, der Porosität der Microspheres, der Temperatur oder dem Abbaumedium.

Der Substitutionsgrad kann über die Stöchiometrie der bei der Acylierungsreaktion eingesetzten Substanzen verändert werden, sollte vorzugsweise jedoch im Rahmen der maximalen Ausbeute (größer 70%) gehalten werden, d.h. ein möglichst großer Prozentsatz der substituierbaren OH-Gruppen am Polymergerüst sollte verestert werden. Ist ein niedrigerer Substitutionsgrad erwünscht, so erniedrigt man entsprechend die Konzentration des Acylierungsagens in Bezug auf das Polymere.

Beim Abbau dieser Polyamide in vivo wird der Ester wieder gespalten und es entstehen die entsprechenden biologisch inaktiven Carbonsäuren bzw. Alkohole und Poly-(hydroxyalkyl)-Aminodicarbonsäure. Dieser Abbau unter physiologischen Bedingungen soll idealerweise ausschließlich körpereigene bzw. in ihrer hohen Bioverträglichkeit bekannte Fragmente erzeugen, die in natürlichen Stoffwechselwegen metabolisiert oder auf Grund ihrer Wasserlöslichkeit durch die Nieren ausgeschieden werden. Zu den bioverträglichen Carbonsäuren bzw. Alkohlen zählen solche mit 6 - 22 C-Atomen im Alkylteil, insbesondere solche mit geradzahliger Anzahl von Kohlenstoffatomen oder die biologisch inaktiven Steroide wie z.B. Cholesterol. Insbesondere wird das Polymer, vorzugsweise das neutrale PHEA zurückgebildet, das auf Grund seiner starken Wechselwirkung mit Wasser ausgezeichnet löslich ist.

Vor allem in diesem Punkt unterscheiden sich die neuen Polymeren von versuchsweise ebenfalls in Implantaten eingesetzten Derivaten von Poly-$\alpha$-Aminosäuren, wie z.B. Poly-$\alpha$-L-Glutaminsäureestern, aus denen durch Biodegradation Polyelektrolyte entstehen, die besonders bei wiederholter Implantation zu toxikologischen und immunologischen Kompliationen Anlaß geben können.

Das Vorliegen von $\alpha$- und $\beta$-Peptidbindungen in D- und L-Form in den bevorzugt eingesetzten $\alpha,\beta$-Poly-(hydroxyalkyl)-DL-Aspartamid-Derivaten verhindert die Ausbildung organisierter Strukturen (z.B. Faltblatt- oder Helixbereiche) im Polymeren, die den Bioabbau in nicht vorhersehbarer Weise beeinflussen.

Die durch die Amidbindungen verursachte partielle Steifigkeit, die durch Wasserstoffbrückenbindungen (N...H...O) hervorgerufen wird, bedingt eine Reihe verarbeitungstechnischer Vorteile dieser erfindungsgemäßen Polymerklasse. Durch Acylierung mit geeigneten reaktiven Carbonsäuren bzw. Alkoholen an der Hydroxyalkylfunktion des Polymers entstehen hydrophobe Polyamide (dann, wenn der Alkylteil mindestens 6 C-Atome aufweist), die sich in einer Vielzahl von organischen Lösungsmitteln lösen und sich aus den Lösungen zu Filmen verarbeiten lassen. Die erfindungsgemäßen Polyamide sind thermoplastisch und eignen sich daher zur Herstellung von Wirkstoffdepotformen nach verschiedenen Methoden, wie z.B. durch Kompression, Extrusion, Fällung, Versprühung, etc.

Aus den erfindungsgemäßen Polyamiden können nach bekannten Methoden implantierbare Partikel, insbesondere Mikrokapseln und Mikrosphären sowie durch Kompaktierung makroskopische Formkörper beliebiger Geometrie, insbesondere Tabletten und Stäbchen hergestellt werden.

Das Polyamid kann beispielsweise mit dem Wirkstoff in einem geeigneten polaren aprotischen Lösungsmittel, beispielsweise Dimethylsulfoxid oder Dimethylacetamid, gelöst werden. Die Lösung wird unter Zugabe eines Emulgators in eine Ölphase (z.B. Paraffin) bei einer Temperatur emulgiert, bei der die Polymerlösung verflüssigt ist. Nach einigen Minuten werden die einzelnen Lösungsmittel-/Polymertröpfchen durch Abkühlen der Emulsion zum Erstarren gebracht. Durch Waschen mit einem geeigneten Lösungsmittel, in dem sich das zur Lösung des Polyamids eingesetzte Lösungsmittel sowie die Ölphase lösen, die Polymertröpfchen jedoch nicht, werden die Polymerkugeln ausgehärtet. Dabei verkleinert sich ihr Volumen, die Form ändert sich nicht.

Die ausgezeichnete Löslichkeit der erfindungsgemäßen Polyamide in organischen Lösungsmittel ermöglicht auch die Bildung von Mikrokugeln durch Vertropfen aus einem Lösungsmittel mit hohem Schmelzpunkt in ein kondensiertes kaltes Gas, z.B. flüssigen Stickstoff, wobei durch das Leidenfrostsche Phänomen absolut runde Partikel entstehen. Das hochschmelzende und mit Wasser mischbare Lösungsmittel wird durch Überführung der Mikrokugeln in Wasser herausgelöst und das Polymere dabei ausgefällt, wobei die Kugelgestalt der Polyamid-Mikrospheres erhalten bleibt.

Besitzt das verwendete organische Lösungsmittel neben einem hohen Schmelzpunkt zugleich einen niedrigen Siedepunkt, so läßt sich dieses Vertropfungsverfahren weiter vereinfachen, indem das Lösungsmittel, z.B. tert. Butanol, direkt mittels Gefriertrocknung der durch Eintropfen in flüssigen Stickstoff gewonnenen Mikrokugeln schonend und ohne Wirkstoffverluste entfernt werden kann.

Besonders vorteilhaft wirkt sich die Löslichkeit der erfindungsgemäßen Polyamide in vielen, auch physiologisch verträglichen Lösungsmitteln, z.B. Alkoholen, bei der Verarbeitung zu Mikrospheres mittels Sprühtrocknung aus. So kann auf den Einsatz von toxikologisch bedenklichen halogenierten Kohlenwasserstoffen, wie sie für die Sprühtrocknung von biodegradablen Polyestern erforderlich sind, bei den erfindungsgemäßen Polyamiden verzichtet werden. Darüber hinaus erlaubt ihre Löslichkeit auch in Alkohol/Wasser-Gemischen die Herstellung von monolithischen wirkstoffhaltigen Mikrokugeln, da hierbei Polymer und Wirkstoff aus molekulardisperser Form versprüht werden können.

Die erfindungsgemäßen Polyamide können auch als Gemische und in Abmischungen mit anderen bioabbaubaren und/oder bioverträglichen Polymeren (z.B. ®Pluronic F68, PHEA, Dextrane, Polyethylenglykole, Hydroxyethylstärke und andere abbaubare oder ausscheidbare Polysaccharide) oder physiologisch unbedenklichen Hilfsstoffen (z.B. Polymerweichmacher) eingesetzt werden.

Abbauversuche in vitro mit den erfindungsgemäßen Polyamiden haben gezeigt, daß die Abbaurate über die funktionellen Seitengruppen kontrolliert gesteuert werden kann.

In den folgenden Beispielen wird die Erfindung im einzelnen beschrieben. Prozentangaben beziehen sich auf das Gewicht, sofern nicht anders angegeben.

**Beispiel 1**

Herstellung von α,β-Poly-(2-cholesteryloxycarbonyloxyethyl)-DL-Aspartamid

3,16 g (20 mmol) α,β-Poly-(2-hydroxyethyl)-DL-Aspartamid (PHEA) werden in 50 ml trockenem N,N-Dimethylformamid (DMF) gelöst. Nach Zugabe von 2 g (25 mmol) Pyridin wird auf 0 °C abgekühlt und unter Rühren innerhalb 10 Minuten 11,23 g (25 mmol) Cholesterylchlorformiat zugegeben. Der Ansatz wird anschließend 2 Stunden bei Raumtemperatur gerührt und dann in 500 ml Ether gegeben. Das ausgefallene Produkt wird abgesaugt, mit Ether, Aceton, Wasser, Aceton und Ether gewaschen und im Vakuum getrocknet. Man erhält 6,5 - 7 g eines bräunlich-weißen Polymers mit einem Substitutionsgrad von ca. 80 %, das sich in DMF und Dichlormethan/Methanol löst und sich oberhalb 250 °C zersetzt.

**Beispiel 2**

Herstellung von α,β-Poly-(2-acetylethyl/2-octanoylethyl)-DL-Aspartamid

3,16 g (20 mmol) PHEA, gelöst in 20 ml DMF, werden mit 3 g (30 mmol) Pyridin versetzt und auf 0 °C abgekühlt. Unter Rühren werden 0,79 g (10 mmol) Acetylchlorid zugetropft und anschließend 30 Minuten bei Raumtemperatur gerührt. Dann wird erneut auf 0 °C abgekühlt und 3,25 g (20 mmol) Octanoylchlorid zugetropft. Anschließend wird noch 2 Stunden bei Raumtemperatur gerührt, in 500 ml Ether gegossen und abgesaugt. Das Produkt wird zur weiteren Reinigung in Methanol gelöst, durch Eintropfen in 10 %ige

wäßrige Essigsäurelösung ausgefällt, abgesaugt, intensiv mit Wasser gewaschen und im Vakuum getrocknet. Man erhält 5 g eines fädenziehenden, fast weißen Polymeren, das sich in DMF, Dichlormethan/Methanol und Methanol löst und oberhalb 180 °C schmilzt.

**Beispiel 3**

Wie in Beispiel 1 angegeben, werden 3,16 g (20 mmol) PHEA mit 4,77 g (25 mmol) Chlorameisensäure-2-cyclohexylethylester zur Reaktion gebracht. Man erhält ca. 5 g eines schwach gelb gefärbten thermoplastischen Polymeren, an dem durch NMR-Spektroskopie keine freien primären Alkoholgruppen mehr nachweisbar sind (Substitutionsgrad größer 90 %).

**Beispiel 4**

Wie in Beispiel 1 angegeben, werden 3,16 g (20 mmol) PHEA mit 9 g (25 mmol) Docosanoylchlorid zur Reaktion gebracht. Das erhaltene Polymer (ca. 8 g) ist thermoplastisch und löst sich in Dichlormethan und Tetrahydrofuran.

**Beispiel 5**

Das zur Reaktion eingesetzte Säurechlorid (4-Chlor-4-oxobuttersäure-n-butylester) wird auf folgendem Wege dargestellt: Bernsteinsäure-monobutylester wird mit überschüssigem Oxalylchlorid und einem Tropfen DMF versetzt, wobei die Reaktion unter Gasentwicklung anspringt.
Man läßt den Ansatz über Nacht unter Feuchtigkeitsausschluß stehen und zieht dann bei 40 °C den Oxyalylchloridüberschuß am Rotationsverdampfer ab. Das Produkt weist IR-Banden bei 1800 $cm^{-1}$ (Säurechlorid) und 1740 $cm^{-1}$ (Ester) von gleicher Intensität auf und wird wegen seiner Zersetzlichkeit ohne weitere Reinigung eingesetzt.

Wie in Beispiel 1 beschrieben, werden 3,16 g (20 mmol) PHEA mit 4,82 g (25 mmol) 4-Chlor-4-oxobuttersäure-n-butylester zur Reaktion gebracht. Im erhaltenen Polymer sind NMR-spektroskopisch keine freien primären Alkoholgruppen mehr nachweisbar.

**Beispiel 6**

3,16 g (20 mmol) PHEA in 30 ml DMF werden mit 4,76 g (25 mmol) Bernsteinsäuremono-n-octylester und 120 mg DMAP (4-Dimethylaminopyridin) versetzt, die Lösung wird auf 0 °C abgekühlt und anschließend eine Lösung von 5,2 g (25 mmol) DCC (Dicyclohexylcarbodiimid) in 20 ml DMF zugetropft. Nach 15 min Rühren bei 0 °C wird über Nacht bei RT weiterreagieren lassen, dann der ausgefallene Dicyclohexylharnstoff abfiltriert und das Filtrat an Rotationsverdampfer auf ~ 20 ml eingeengt. Das Polymer wird durch Eintropfen der DMF-Lösung in 500 ml Ether ausgefällt.

**Beispiel 7**

Analog Beispiel 1 werden 3,16 g (20 mmol) PHEA mit 6,15 g (25 mmol) Tetradecanoylchlorid zur Reaktion gebracht. Das erhaltene schwach bräunliche Polymer (ca. 5,1 g) ist thermoplastisch und löst sich in Dichlormethan und Tetrahydrofuran.

**Beispiel 8**

Analog Beispiel 1 werden 3,16 g (20 mmol) PHEA mit 4,05 g (25 mmol) Oxtanoylchlorid zur Reaktion gebracht. Das erhaltene gelblich-weiße Polymer (ca. 4,8 g) ist thermoplastisch und löst sich in Dichlormethan und Tetrahydrofuran.

**Beispiel 9**

Analog Beispiel 2 werden 3,16 g (20 mmol) PHEA mit 0,91 g (10 mmol) Methoxycarbonylchlorid und 3,24 g (20 mmol) Octanoylchlorid versetzt. Das erhaltene gelblich-weiße Polymer (ca. 5,0 g) ist thermoplastisch und löst sich in Dichlormethan und Tetrahydrofuran.

**Beispiel 10**

Herstellung von Microspheres

40 mg $C_{14}$-PHEA aus Beispiel 7 werden in 1 ml Methylenchlorid/Methanol (Volumenanteil 50/1) gelöst. Die Lösung wird mit 10 mg Buserelin versetzt, das mit Ultraschall dispergiert wird. Die Dispersion wird unter Rühren (800 Upm) in ein Becherglas mit 60 ml 0,1 Gew.-%iger, wäßriger Polyvinylalkohol-Lösung (®Mowiol 28-99) eingebracht, die mit 0,3 ml Methylenchlorid/Methanol (50/1) gesättigt ist.

Nach 5 Minuten wird der Inhalt in ein Becherglas mit 200 ml Wasser gegeben und 30 Minuten gerührt (200 Upm). Das überstehende Wasser wird abdekantiert und die Microspheres werden lyophilisiert (Durchmesser nach Lyophilisation: 20-90 µm).

**Beispiel 11**

Herstellung von Microspheres

44 mg $C_2$-$C_8$-PHEA aus Beispiel 2 werden in 100 ml Dimethylsulfoxid gelöst und danach mit 1000 µl Methylenchlorid versetzt.

Die Lösung wird unter Rühren (800 Upm) in ein Becherglas mit 60 ml 0,1 Gew.-%iger, wäßriger Lösung von Carboxymethylcellulose (®Serva, 300 cps) bei 4°C eingebracht, die mit 0,3 ml Methylenchlorid gesättigt ist.

Nach 5 Minuten wird der Inhalt gemäß Beispiel 10 aufgearbeitet.

**Beispiel 12**

Herstellung von Microspheres

80 mg $C_8$-PHEA aus Beispiel 8 werden bei 50°C in 1 ml Dimethylsulfoxid gelöst und mit 20 mg Hydroxypropylcellulose (®Klucel M.) versetzt. Die Lösung der beiden Polymeren wird mit einer Kanüle (Einwegspritze, Kanülendurchmesser außen 0,6 mm) in eine Vorlage von flüssigem Stickstoff (100 ml) eingetropft.

Die entstandenen Mikrospheres werden in 200 ml Wasser überführt und 2 Stunden von restlichem Lösungsmittel extrahiert. Überschüssiges Wasser wird abdekantiert und die Microspheres werden lyophilisiert (Duchmesser nach Lyophilisation 1-2 mm, Erweichungsbereich 190-210°C).

**Beispiel 12a**

Herstellung von Mikrospheres durch direktes Gefriersprühen aus tert. Butanol

320 mg $C_6$-PHEA werden in wenig Ethanol angequollen und in 3,6 g tert. Butanol gelöst. 80 mg mikronisiertes Buserelin werden druch Ultrabeschallung in dieser Lösung dispergiert und die Mischung durch eine feine Düse in flüssigen Stickstoff vertropft. Der flüssige Stickstoff wird dekantiert und die Mikrokugeln durch sofortige Gefriertrocknung vom Lösungsmittel befreit.

**Beispiel 12b**

Herstellung von Mikrospheres durch Sprühtrocknung

400 mg Polyamid nach Beispiel 5 wird in 8 ml 90 %igem Ethanol gelöst und mit einer Lösung von 100 mg PHEA und 100 mg Buserelin in 1 ml Wasser vereinigt. Das Gemisch wird in einem Sprühtrockner zu Mikrokugeln versprüht.

**Beispiel 13** Polymerabbau

Es wird die Wasserresorption (in Gew.-%) verschieden substituierter Polyhydroxyethylaspartamide nach 74 h Lagerung bei 92% relativer Luftfeuchte bestimmt sowie die Dauer der Hydrolyse der Alkylester-/Alkylcarbonat-Seitengruppen (in Stunden) bis zur vollständigen Solubilisierung von je 100 mg Polymerpulver in 100 ml wäßriger NaOH (pH 13):

| Polymer | aus Beispiel | Wasserresorption % | Solubilisierung h |
|---|---|---|---|
| $C_8$-PHEA | 8 | 25 | 3 |
| $C_{14}$-PHEA | 7 | 8 | 200 |
| $C_2/C_8$-PHEA | 2 | 12 | 24 |
| $C_1C/C_8$-PHEA | 9 | 80 | 1 |

**Beispiel 14** Polymerabbau

3 Proben von jeweils 500 mg Polymer werden in je 30 ml einer Phosphat-Pufferlösung aus 0,00205 mol $Na_2HPO_4$ und 0,0045 mol $NaH_2PO_4$ (pH 7,4) inkubiert und in verschlossenen Glasflaschen (50 ml) bei 37° gerührt.

Der Phosphatpuffer wird mit 0,0078 mol $NaN_3$ gegen mikrobiellen Befall stabilisiert und nach jeweils 7 Tagen in seinem pH korrigiert.

Über einen Zeitraum von 150 Tagen werden die Gewichtsabnahmen der Polymerproben gemessen: die Pufferlösung mit inkubiertem Polymer wird über eine tarierte Glasfritte filtriert, der Rückstand 24 h im Vakuum über Phosphorpentaoxid getrocknet und die Gewichtabnahme bestimmt.

| Polymer | aus Beispiel | Gewichtsabnahme (%) | | |
|---|---|---|---|---|
| | | 14 Tage | 70 Tage | 150 Tage |
| $C_8$-PHEA | 8 | 5 | 22 | 84 |
| $C_{14}$-PHEA | 7 | 0 | 5 | 21 |
| $C_2/C_8$-PHEA | 2 | 0 | 14 | 43 |
| $C_{10}/C_8$-PHEA | 9 | 11 | 65 | 100 |

**Beispiel 15**

Herstellung von stäbchenförmigen Implantaten ("rods")

Ein inniges Gemisch aus pulverförmigen Polymeren, Additiven und Wirkstoffe(n) wird in einer geeigneten Vorrichtung, z.B. einem Extruder für Thermoplasten, über den Erweichungspunkt erhitzt, wobei eine verformbare Masse entsteht. Durch Kneten werden Additive und Wirkstoff(e) im erweichten Polymer homogen dispergiert und die erhaltene Polymer/Wirkstoff-Suspension durch eine Düse geeigneten Durchmessers (> 0,5 mm) gepreßt. Beim Abkühlen erstarrt der Strang des extrudierten Polymer/Wirkstoff-Suspension zu einem festen stäbchenförmigen Aggregat, dessen Wirkstoffgehalt von seiner Länge und seinem Durchmesser bestimmt ist.

**Beispiel 16**

Wirkstoffabgabe (Release von Buserelin)

Analog Beispiel 10 werden Microspheres mit einer Zusammensetzung von
88 Gew.-% LEMF
6 Gew.-% $C_2$-$C_8$-PHEA aus Beispiel 2
6 Gew.-% Buserelin
hergestellt. LEMF = Polylysinethyl/methylesterfumaramid ist Gegenstand der deutschen Patentanmeldung Nr. P 36 16 320.1 (Beispiel 8) und wird beispielsweise hergestellt durch Polykondensation von Fumarsäurechlorid, Lysinmethylesterdihydrochlorid und Lysinethylesterdihydrochlorid.

Die Wirkstoffabgabe Buserelin-Release) wird in einer Pufferlösung (2,91 g $Na_2HPO_4$, 0,540 g $NaH_2PO_4$, 0,4 g $NaN_3$, 6,328 g NaCl und 2,52 g $NaHCO_3$ auf 1 l Wasser) UV-spektroskopisch gemessen. In Fig. 1 ist der gesamte abgegebene Anteil von Buserelin (in Prozent) als Funktion der Zeit aufgetragen.

## Patentansprüche

**1.** Poly-(hydroxyalkyl)-Aminodicarbonsäure-Derivate der Formel I

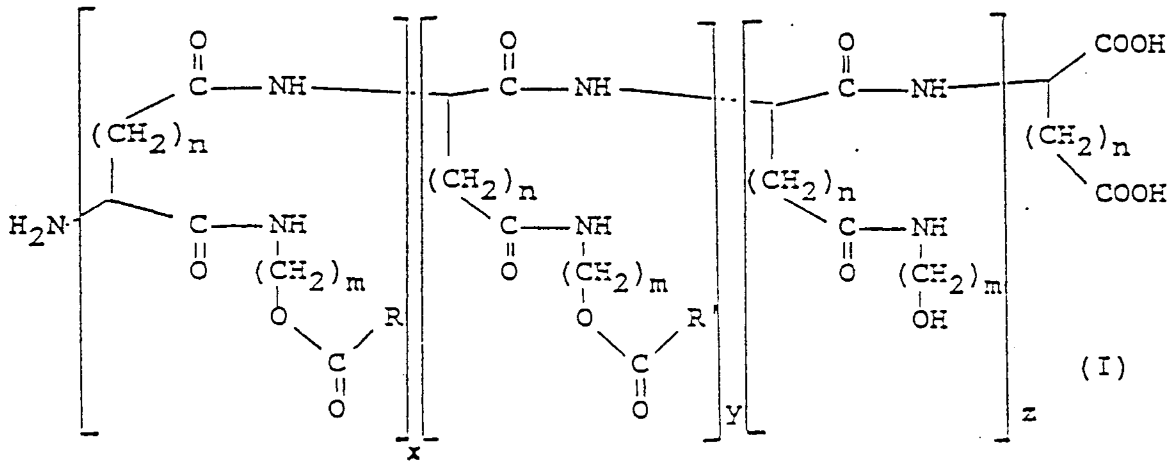

(I)

in der n 1 oder 2, m 2 bis 6, x und y 1 bis 400 und z 0 bis 40 sind, und die Summe von x, y und z 2 bis 840 ist und der Anteil der Hydroxyalkylamino-dicarbonsäureamid-Monomereinheit bezogen auf den molaren Anteil der acylierten Aminodicarbonsäureamide nicht mehr als 30 % ist und in der die Reste R und R' gleich oder unabhängig voneinander verschieden sind und gesättigte verzweigte oder unverzweigte Alkyl-, Cycloalkyl-, Alkoxy- oder Cycloalkyloxy-Gruppen mit insgesamt 1 - 22 C-Atomen im Alkylteil, wobei der Alkylteil gegebenenfalls durch eine Carbonyloxy-Gruppe unterbrochen sein kann, oder biologisch inaktive Steroidalkohole, gebunden über ihre Hydroxylgruppen, bedeuten, wobei die in eckige Klammern gesetzten Monomereinheiten statistisch im Polymeren verteilt sind.

**2.** Poly-(hydroxyalkyl)-Aminodicarbonsäure-Derivate gemäß Formel I nach Anspruch 1, in der n 1 und m 2 und die Reste R und R' gleich oder unabhängig voneinander verschieden sind und unverzweigte Alkyl- oder Alkoxy-Gruppen mit 1 - 22 C-Atomen im Alkylteil, wobei der Alkylteil gegebenenfalls durch eine Carbonyloxy-Gruppe unterbrochen sein kann, bedeuten.

**3.** Poly-(hydroxyalkyl)-aminodicarbonsäure-Derivate gemäß Formel I nach Anspruch 1, in der n 1 und m 2 ist und R eine Methyl- oder Methoxy-Gruppe und R' eine Pentyl- oder Butyloxycarbonylethyl-Gruppe ist.

**4.** Verfahren zur Herstellung von Poly-(hydroxyalkyl)-Aminodicarbonsäure-Derivatender Formel I, dadurch gekennzeichnet, daß man eine Poly-(hydroxyalkyl)-aminodicarbonsäure der Formel IIa

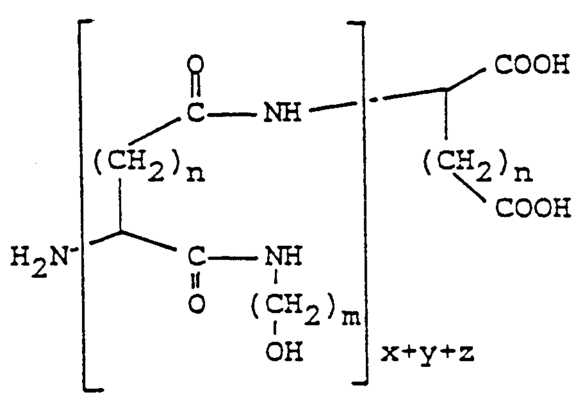

(IIa)

mit einer oder mehreren verschiedenen Verbindungen der Formel V und/oder VI

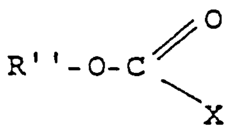 (V) 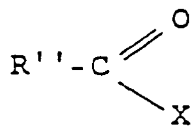 (VI)

umsetzt, wobei n, m, x, y und z, die in Anspruch 1 zu Formel I angegebenen Bedeutungen haben, wobei R" für eine gesättigte verzweigte oder unverzweigte Alkyl-, Cycloalkyl-, Alkoxy- oder Cycloalkyloxy- Gruppe mit insgesamt 1 - 22 C-Atomen im Alkylteil steht, wobei der Alkylteil gegebenenfalls durch eine Carbonyloxy-Gruppe unterbrochen sein kann, oder einen biologisch inaktiven Steroidalkohol, gebunden über die Hydroxylgruppe, bedeutet und X Chlor, Brom, Jod oder Hydroxyl ist.

**5.** Verwendung eines Polyamids nach Anspruch 1, 2 oder 3 zur Herstellung von biologisch abbaubaren Wirkstoffdepotzubereitungen mit kontrollierter Wirkstoffabgabe.

**6.** Verwendung eines Polyamids nach Anspruch 1, 2 oder 3 zum Einkapseln von biologisch aktiven Substanzen.

**7.** Verwendung eines Polyamids nach Anspruch 1, 2 oder 3 in Abmischungen mit anderen bioabbaubaren und/oder bioverträglichen Polyamiden oder physiologisch unbedenklichen Hilfsstoffen zur Herstellung von biologisch abbaubaren Wirkstoffdepotzubereitungen mit kontrollierter Wirkstoffabgabe.

## Claims

**1.** Poly(hydroxyalkyl)amino dicarboxylic acid derivatives of the formula I

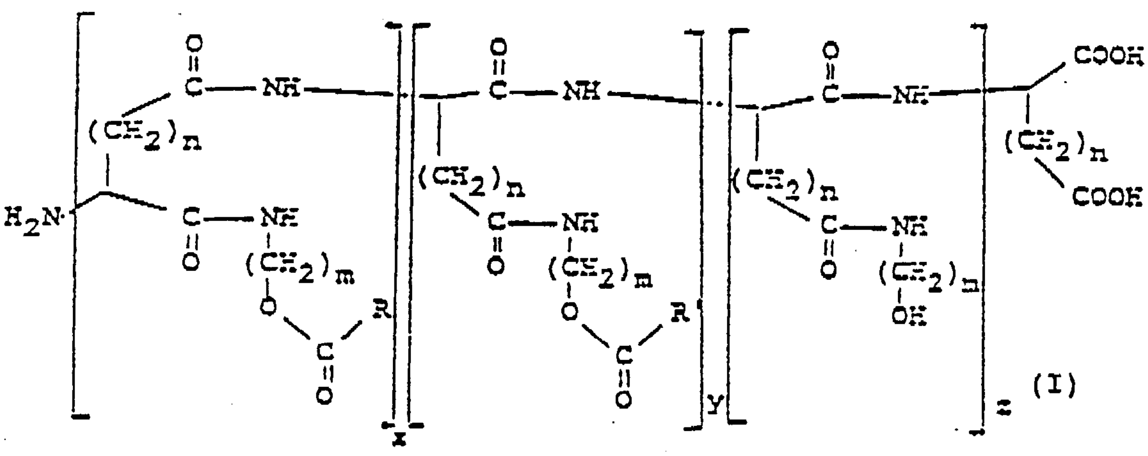

(I)

in which n is 1 or 2, m is 2 to 6, x and y are 1 to 400, z is 0 to 40, and the sum of x, y and z is 2 to 840 and the proportion of the hydroxyalkylamino dicarboxamide monomer unit, based on the molar proportion of the acylated amino dicarboxamides, is not more than 30%, and in which the radicals R and R' are identical or, independently of one another, different and denote saturated branched or unbranched alkyl, cycloalkyl, alkoxy or cycloalkyloxy groups having a total of 1 - 22 carbon atoms in the alkyl moiety, it being possible for the alkyl moiety optionally to be interrupted by a carbonyloxy group, or biologically inactive steroid alcohols bonded via their hydroxyl groups, there being random distribution in the polymer of the monomer units placed in square brackets.

**2.** Poly(hydroxyalkyl)amino dicarboxylic acid derivatives of the formula I as claimed in claim 1, in which n is 1 and m is 2, and the radicals R and R' are identical or, independently of one another, different and denote unbranched alkyl or alkoxy groups having 1 - 22 carbon atoms in the alkyl moiety, it being possible for the alkyl moiety optionally to be interrupted by a carbonyloxy group.

3. Poly(hydroxyalkyl)amino dicarboxylic acid derivatives of the formula I as claimed in claim 1, in which n is 1 and m is 2, and R is a methyl or methoxy group, and R' is a pentyl- or butyloxycarbonylethyl group.

4. A process for the preparation of poly(hydroxyalkyl)amino dicarboxylic acid derivatives of the formula I, which comprises reaction of a poly(hydroxyalkyl)amino dicarboxylic acid of the formula IIa

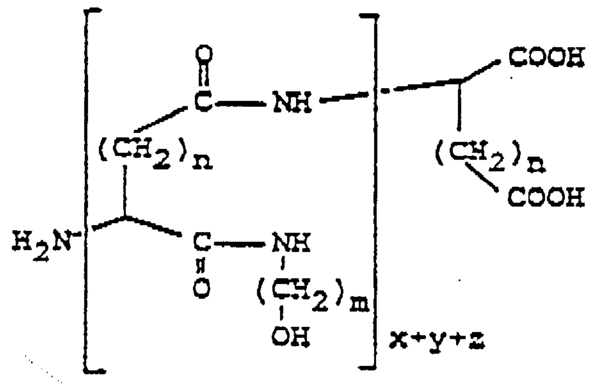

(IIa)

with one or more different compounds of the formula V and/or VI

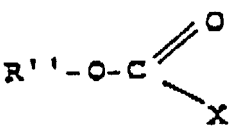

(V)

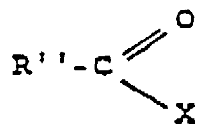

(VI),

n, m, x, y and z having the meanings indicated for formula I in claim 1, R'' being a saturated branched or unbranched alkyl, cycloalkyl, alkoxy or cycloalkyloxy group having a total of 1 - 22 carbon atoms in the alkyl moiety, it being possible for the alkyl moiety optionally to be interrupted by a carbonyloxy group, or a biologically inactive steroid alcohol bonded via the hydroxyl group, and X being chlorine, bromine, iodine or hydroxyl.

5. The use of a polyamide as claimed in claim 1, 2 or 3 for the preparation of biodegradable active ingredient depot formulations with controlled delivery of active ingredient.

6. The use of a polyamide as claimed in claim 1, 2 or 3 for the encapsulation of biologically active substances.

7. The use of a polyamide as claimed in claim 1, 2 or 3 in mixtures with other biodegradable and/or biocompatible polyamides or physiologically acceptable auxiliaries for the preparation of biodegradable active ingredient depot formulations with controlled delivery of active ingredient.

## Revendications

1. Dérivés d'acides poly-(hydroxyalkyl)-aminodicarboxyliques de formule I

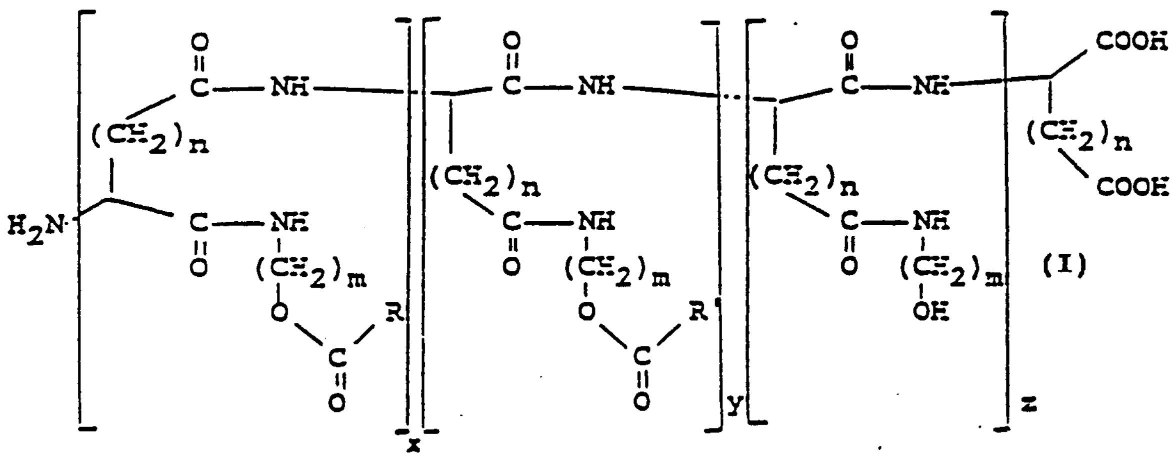

(I)

dans laquelle n est 1 ou 2, m va de 2 à 6, x et y vont de 1 à 400, et z va de 0 à 40, et la somme de x, y et z va de 2 à 840, et la proportion du motif monomère hydroxyalkylamino-dicarboxamide, par rapport à la fraction molaire des amino-dicarboxamides acylés n'excède pas 30 %, et dans laquelle les radicaux R et R' sont identiques ou différents, indépendamment les uns des autres, et représentent des groupes alkyle, cycloalkyle, alcoxy ou cycloalkyloxy saturés, ramifiés ou non ramifiés, ayant au total de 1 à 22 atomes de carbone dans le fragment alkyle, le fragment alkyle pouvant éventuellement être interrompu par un groupe carbonyloxy, ou des stéroïde-alcools biologiquement inactifs, liés par l'intermédiaire de leurs groupes hydroxy, les motifs monomères mis entre crochets étant répartis statistiquement dans le polymère.

2. Dérivés d'acides poly-(hydroxyalkyl)-amino-dicarboxyliques de formule I selon la revendication 1, dans lesquels n est 1 et m est 2 et les radicaux R et R' sont identiques ou différents, indépendamment les uns des autres, et représentent des groupes alkyle ou alcoxy non ramifiés ayant de 1 à 22 atomes de carbone dans le fragment alkyle, le fragment alkyle pouvant éventuellement être interrompu par un groupe carbonyloxy.

3. Dérivés d'acides poly-(hydroxyalkyl)-amino-dicarboxyliques de formule I selon la revendication 1, dans lesquels n est 1 et m est 2, et R est le groupe méthyle ou méthoxy, et R' est le groupe pentyle ou butyloxycarbonyléthyle.

4. Procédé pour la préparation de dérivés d'acides poly-(hydroxyalkyl)-aminodicarboxyliques de formule I, caractérisé en ce que l'on fait réagir un acide polyhydroxyalkyl)-aminodicarboxylique de formule IIa

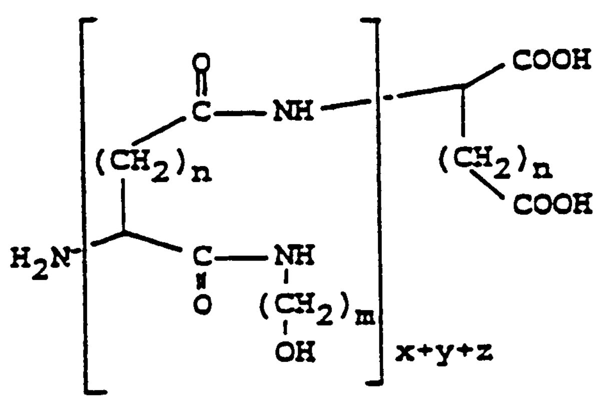

(IIa)

avec un ou plusieurs composés différents, de formule V et/ou de formule VI

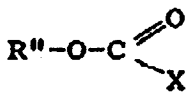 (V) 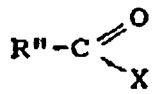 (VI)

n, m, x, y et z ayant les significations données à propos de la formule I dans la revendication 1, R'' représentant un groupe alkyle, cycloalkyle, alcoxy ou cycloalkyloxy saturé, ramifié ou non ramifié, ayant au total de 1 à 22 atomes de carbone dans le fragment alkyle, le fragment alkyle pouvant éventuellement être interrompu par un groupe carbonyloxy, ou un stéroïde-alcool biologiquement inactif, lié par l'intermédiaire du groupe hydroxy, et X étant un atome de chlore, brome ou iode, ou le groupe hydroxy.

**5.** Utilisation d'un polyamide selon la revendication 1, 2 ou 3, pour la préparation de compositions retard biologiquement dégradables de substances actives, à libération programmée de la substance active.

**6.** Utilisation d'un polyamide salon la revendication 1, 2 ou 3, pour l'encapsulation de substances biologiquement actives.

**7.** Utilisation d'un polyamide selon la revendication 1, 2 ou 3, dans des mélanges avec d'autres polyamides biodégradables et/ou biocompatibles ou des adjuvants physiologiquement acceptables, pour la préparation de compositions retard biologiquement dégradables de substances actives, à libération programmée de la substance active.

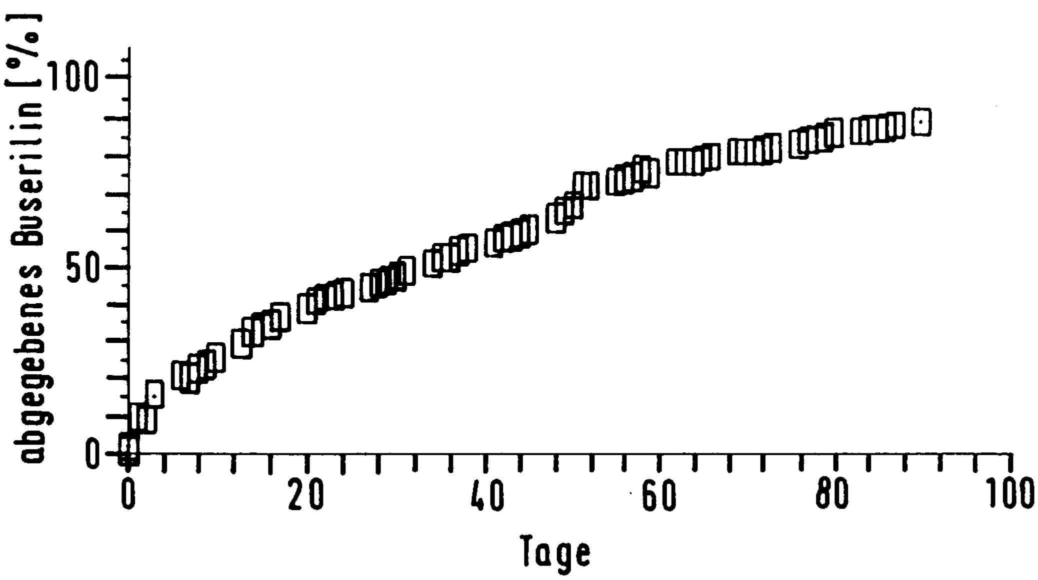


Fig. 1